# FASCICULE DE BREVET EUROPEEN

(11) **EP 0 246 158 B1**
(45) Date de publication et mention de la délivrance du brevet: **07.01.1993**
(21) Numéro de dépôt: 87401083.8
(22) Date de dépôt: 14.05.1987
(51) Int. Cl.: A61M 5/14

(54) **Dispositif externe pour injecter un médicament**
Äusserliche Arzneiinjektionsvorrichtung
External device for medical injection

(30) Priorité: 14.05.1986 FR 8606940; 10.07.1986 FR 8610105
(43) Date de publication de la demande: 19.11.1987
(73) Titulaire: Buffet, Jacques, F-93250 Villemomble (FR); Buffet, Jean-Paul, F-93340 Le Raincy (FR); COMPAGNIE FINANCIERE SAINT-NICOLAS, F-93340 Le Raincy (FR)
(72) Inventeur: Piani, Jean, F-20000 Ajaccio (FR); Paravisini, François, F-20000 Ajaccio (FR)
(74) Mandataire: Derambure, Christian

(56) Documents cités:
- WO-A-82/00589
- WO-A-85/01443
- DE-C- 600 202
- GB-A- 2 109 690
- US-A- 3 384 080
- US-A- 3 709 636
- US-A- 3 886 938
- US-A- 3 989 913
- US-A- 3 989 913

## Description

L'invention concerne un dispositif externe pour injecter un médicament.

Plus précisément, elle concerne un dispositif pour l'injection, notamment de l'insuline, dans le corps humain du type comprenant une entrée, une sortie, des moyens mobiles de transfert interposés entre l'entrée et la sortie comprenant un organe mobile d'entraînement, des premiers moyens d'entraînement, mobiles, associés à l'organe mobile et assurant son déplacement, et une source d'énergie méanique associée aux premiers moyens d'entraînement et propres à délivrer la puissance énergétique nécessaire à leur fonctionnement .

De nombreux dispositifs de ce type sont connus qui présentent chacun des inconvénients que l'invention résout simultanément.

Les brevets français FR 2 282 912, FR 2 495 942, FR 2 348 709, le brevet allemand DE 600202, le brevet américain US 3 709 636 décrivent des systèmes d'injection complexes, volumineux et actionnés par des moteurs électriques qui consomment beaucoup d'énergie.

Les demandes de brevet PCT WO 85 01443 comme le brevet américain US 3 384 080 décrivent des systèmes actionnés mécaniquement mais qui ne permettent pas l'injection de doses instantanées.

La demande de brevet anglaise GB 2 109 690 décrit une seringue particulièrement destinée à l'injection d'insuline. Son actionnement est manuel.

On connaît du document WO-A-82/00589 un dispositif externe conforme au préambule de la première revendication et comportant un mécanisme d'entraînement entièrement mécanique pour l'injection de doses continues en régime permanent.

L'invention a donc pour objet un tel dispositif présentant en outre les éléments de la partie caractéristique de la première revendication.

Des modes préférés et avantageux de réalisation de l'invention font l'objet des sous-revendications.

Les autres caractéristiques de l'invention résulteront de la description qui suivra en référence aux dessins annexés dans lesquels :
- La figure 1 est une vue schématique en coupe par un plan axial illustrant le dispositif selon le premier mode de réalisation de l'invention, les deux parties du dispositif étant dissociées l'une de l'autre pour une meilleure compréhension.
- La figure 2 est une vue schématique de dessus du dispositif selon l'invention afin de montrer la coopération des deux parties du boîtier.
- La figure 3 est une vue schématique en section transversale selon la ligne III-III de la figure 1.
- La figure 4 est une vue schématique à plus grande échelle du réservoir et des moyens de rappel du cathéter.
- La figure 5 est une vue schématique à plus grande échelle des moyens d'association de la vis sans fin et du piston.
- La figure 6 est une vue schématique en coupe par un plan axial illustrant la seconde partie d'un autre mode de réalisation de l'invention.
- La figure 7 est une vue schématique illustrant un mode de réalisation des moyens limiteurs de couple selon l'invention.
- La figure 8 est une vue schématique illustrant l'utilisation générale connue en soi d'une pompe d'injection de médicaments.
- La figure 9 est une vue schématique en élévation du dispositif selon le deuxième mode de réalisation de l'invention.
- La figure 10 est une vue schématique en élévation des moyens de régulation du dispositif selon le deuxième mode de réalisation de l'invention.
- La figure 11 est une vue schématique sous forme de bloc diagramme des moyens de commande des moyens de régulation selon le deuxième mode de réalisation de l'invention.

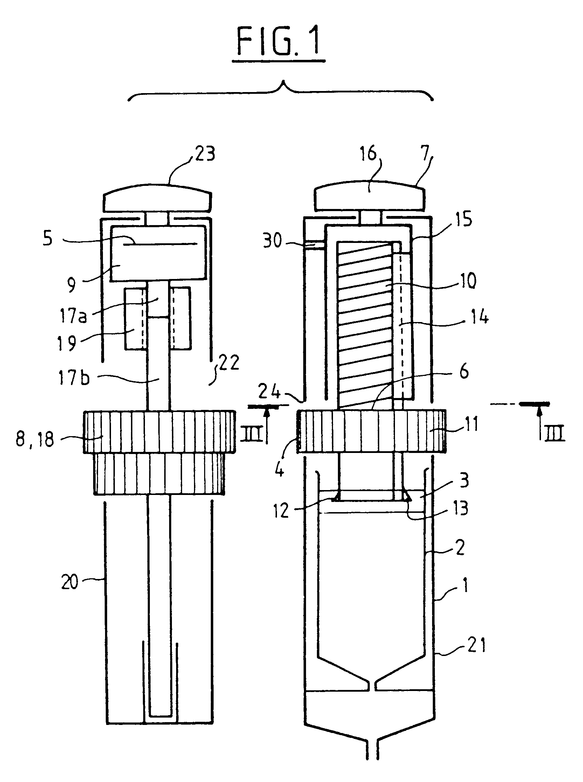

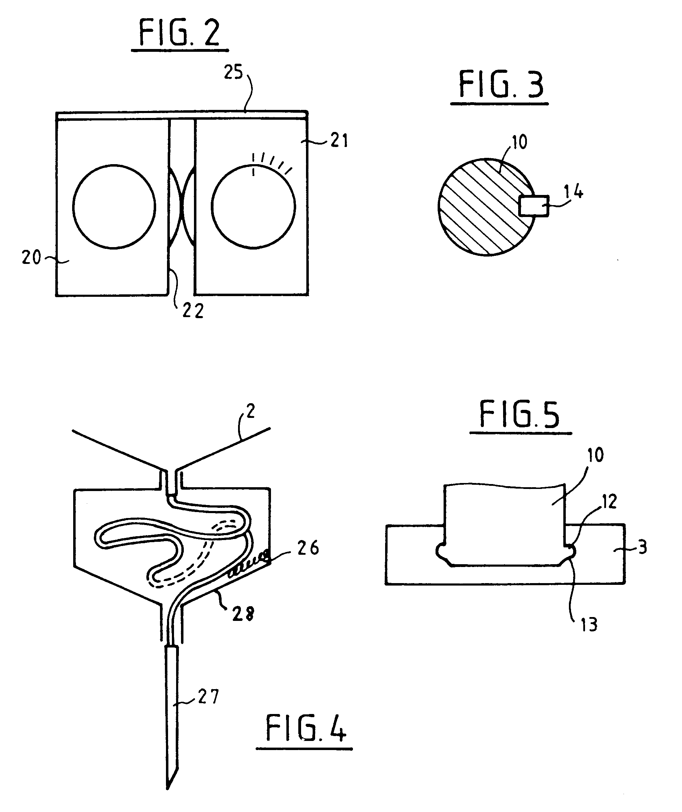

On décrira tout d'abord un premier mode de réalisation de l'invention en référence aux figures 1 à 7.

L'invention concerne un dispositif externe pour injecter un médicament ou autre, notamment de l'insuline, dans le corps humain, du type comportant un boîtier 1 portable (représenté seulement partiellement et schématiquement), une seringue 2 ayant un piston 3 montée amovible dans le boitier 1, des moyens d'entraînement 4 du piston 3 à coulissement axial comprenant d'une part des moyens de stockage d'énergie 5 et des moyens automatiques d'entraînement linéaire 6 du piston 3 activés par l'énergie contenue dans les moyens de stockage d'énergie 5 et d'autre part des moyens manuels d'entraînement linéaire 7 à molette d'actionnement 16.

Le dispositif selon l'invention permet simultanément l'administration de doses instantanées ou bolus grâce aux moyens manuels 7 comme on le verra ultérieurement et d'autre part l'administration de doses continues à débit constant ou débit de base grâce aux moyens de stockage d'énergie 5 et aux moyens automatiques d'entraînement 6.

Selon l'invention, les moyens de stockage d'énergie 5 sont mécaniques, intégrés au boîtier 1 et sont notamment constitués de moyens élastiques. Les moyens automatiques d'entraînement linéaire 6 sont également mécaniques. Les moyens automatiques d'entraînement sont constitués de moyens 9 de régulation du mouvement engendré par les moyens de stockage d'énergie 5, notamment par la détente des moyens élastiques 5, et de moyens mécaniques 8 de transmission du mouvement au piston 3.

Les moyens de stockage d'énergie 5 ont seulement pour fonction d'assurer la mise en mouvement des moyens moteurs automatiques d'entraînement linéaire 6 pour l'administration du débit de base tandis que, comme on l'a dit précédemment, l'administration du bolus est assurée seulement par l'actionnement des moyens manuels d'entraînement linéaire 7.

Préférentiellement, les moyens de stockage d'énergie 5 sont constitués par un ressort notamment un ressort spiralé tandis que les moyens 9 de régulation comprennent un mouvement mécanique d'horlogerie 9, à échappement et balancier horizontal entraîné par le ressort spiral.

Les moyens d'entraînement linéaire 4 comprennent également une vis sans fin 10 coopérant avec un écrou 11. L'écrou 11 est mobile en rotation et bloqué axialement notamment par le boîtier 1. Il forme extérieurement un pignon mené. La vis sans fin 10 est principalement bloquée en rotation sous réserve de ce qui est mentionné ci-dessous et mobile axialement par rapport à un fourreau 15 monté pivotant dans le boîtier 1. Elle se termine, du côté de la seringue 2 par une saillie périphérique 12 coopérant de façon amovible et à force avec un logement périphérique 13 du piston 3. La saillie 12 et le logement 13 constituent des moyens 12, 13 d'association rigide amovible de la vis 10 au piston 3.

La vis sans fin 10 est bloquée en rotation par rapport au fourreau 15 par au moins une clavette 14 s'étendant axialement. La clavette 14 est portée par le fourreau 15 monté pivotant dans le boîtier 1 et auquel est fixé la mollette d'actionnement manuel 16.

Le fourreau 15 est bloqué en rotation par rapport au boîtier 1, de préférence dans un seul sens de rotation, par des moyens 30 de blocage à cliquet anti-retour. Il est ainsi bloqué dans un sens et libre dans l'autre sens. Préférentiellement, les moyens 8 de transmission comportent des organes amovibles et remplaçables de réglage de la vitesse.

Les moyens 8 de transmission comportent en outre un axe 17 sur lequel est monté calé au moins un pignon moteur 18 coopérant avec l'écrou formant pignon mené 11. L'axe 17 est entraîné en rotation par le mouvement mécanique d'horlogerie 9.

Préférentiellement, des moyens limiteurs de couple 19 sont interposés sur l'axe 17 en deux parties 17a, 17b, entre le pignon moteur 18 et le mouvement d'horlogerie 9. Ces moyens limiteurs de couple 19 permettent d'éviter la détérioration du cathéter 27 notamment lorsque celui-ci est bouché, par application d'une trop grande pression.

Les organes amovibles et remplaçables de réglage de la vitesse des moyens 8 de transmission sont constitués par exemple par plusieurs pignons moteurs 18 de différents diamètres susceptibles de coopérer chacun avec le pignon mené 11 et associés rigidement mais de façon amovible à l'axe 17 ou à une partie 17a de cet axe. Par exemple ces pignons noteurs 18 comportent un alésage axial muni d'une denture radiale (c'est-à-dire pour laquelle les dents ont leur hauteur radiale) susceptible de coopérence avec une denture radiale étendue le long de l'axe 17 et solidaire de cet axe 17 ou de la partie 17a.

Le boîtier 1 comporte préférentiellement deux parties 20, 21, la première partie 20 dans laquelle est monté l'axe 17, le (ou les) pignon(s) 18, les moyens limiteurs de coupe 19 et le mouvement d'horlogerie 9. Cette première partie 20 comporte une fenêtre latérale 22 d'accès aux dents du (ou des) pignon(s) 18 de laquelle saille une partie de la périphérie du (ou des) pignon(s) 18 pour permettre sa (leur) coopération avec l'autre pignon 11. Enfin, un bouton remontoir 23 est placé à l'extérieur de la première partie 20 pour permettre de remonter le ressort 5 du mouvement d'horlogerie 9.

La seconde partie 21 comporte d'un côté un logement dans lequel est monté la seringue 2 et de l'autre côté, le fourreau 15 avec sa clavette 14, la vis sans fin 10 coopérant avec l'écrou 11 formant pignon mené. L'écrou 11 formant pignon mené saille d'une fenêtre 24 de cette seconde partie 21 pour permettre sa coopération avec le (ou les) pignon(s) 18.

Des moyens d'association amovibles 25 représentés seulement schématiquement permettent d'associer l'un à l'autre de façon rigide mais amovible les deux parties 20 et 21 de manière que les pignons 11, 18 coopèrent l'un avec l'autre.

Selon une autre caractéristique, il est prévu un logement d'extrémité 28 de la seconde partie 21 formant réservoir pour le cathéter 27, et des moyens de rappel 26 du cathéter 27 associé à la seringue 2 dans ce logement 28. Ces moyens de rappel 26 peuvent être constitués d'un ou plusieurs ressorts. En variante, ils sont constitués d'une molette à grand pas de vis intérieur montée à la sortie du logement 28, qui autorise l'extraction du cathéter 27 hors du logement 28, mais qui force le cathéter en sens contraire dans le logement 28 lorqu'on la tourne. Ces moyens 26 de rappel peuvent aussi être constitués d'un ressort spiral de rappel d'une bobine autour de laquelle le cathéter 27 s'enroule. Le logement 28 et les moyens 26 de rappel permettent d'éviter qu'une trop grande longueur de cathéter 27 ne reste sortie, tout en fournissant la longueur désirée de cathéter 27.

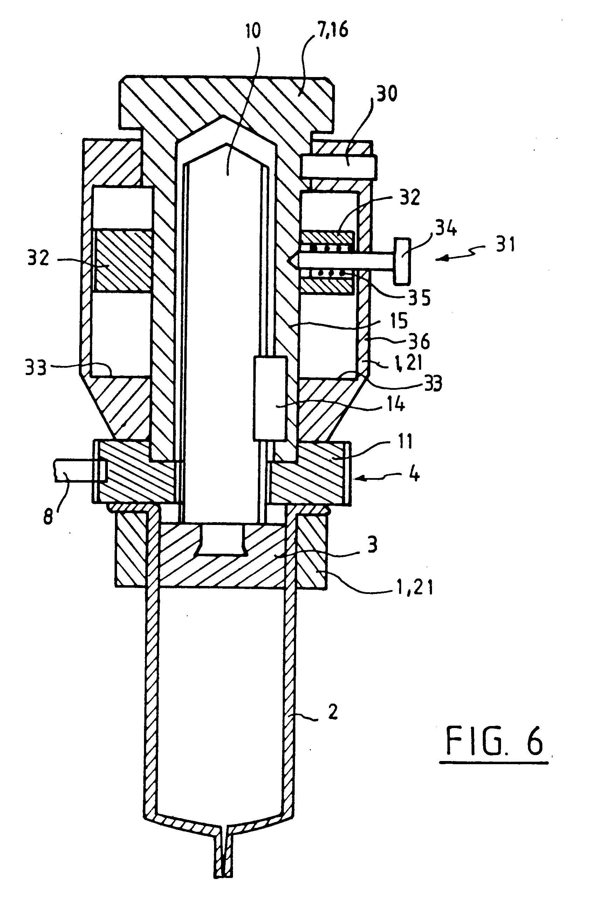

On a représenté en figure 6 un autre mode de réalisation de la partie 21 du dispositif selon l'invention portant la seringue.

Les moyens 30 de blocage du fourreau 15 par rapport au boîtier, comportent au moins un cliquet 30 à ressort coopérant avec des dents périphériques radiales externes du fourreau 15 pour le bloquer en rotation dans un sens de rotation S1. Par contre ces moyens 30 de blocage laissent le fourreau 15 libre de tourner dans l'autre sens 52, lorsqu'on actionne manuellement la molette 16. Dans ce cas les bruits occasionnés par le cliquet 30 au passage de chaque dent du fourreau 15 permet à l'utilisateur de contrôler la rotation qu'il impose à la molette 16. Des numérotations ou graduations peuvent être prévues au voisinnage des dents sur le fourreau 15 ou sur la molette 16 pour permettre le contrôle visuel de la rotation par l'utilisateur. Les moyens 30 de blocage constituent donc également des moyens de dosage fin de la dose injectée manuellement.

Par ailleurs, le dispositif tel que représenté en figure 6 comporte également des moyens 31 limiteurs de la dose manuelle injectée, constitués d'une bague 32 extérieure au fourreau 15 et coopérant avec un filetage extérieur à grand pas de ce fourreau 15, de façon que la rotation du fourreau 15 dans le sens S2 induise le coulissement de la bague 32 vers une butée 33 du boîtier 1, simultanément et proportionellement au déplacement axial de la vis 10. Un pointeau 34 dont la pointe est rappelée par un ressort 35 dans les filets extérieurs du fourreau 15 permet de positionner la bague à la hauteur correspondant à la dose désirée. Le pointeau 34 traverse et coulisse dans une rainure 36 du boitier 1 qui comporte avantageusement des graduations ou repères. Pour injecter une dose manuellement, l'utilisateur positionnera donc la bague 32 en tirant le pointeau 34 hors des filets du fourreau 15 puis en faisant coulisser la bague 32 de façon à placer le pointeau 34 en face du repère correspondant sur le boîtier à la dose désirée ou à la dose maximale. Ensuite l'utilisateur relâche le pointeau 34 qui coopére à nouveau avec les filets du fourreau 15, puis tourne la molette 16 dans le sens S2. La rotation sera stoppée quand la bague 32 arrive contre la butée 33, ce qui empêche l'injection d'une dose trop importante manuellement.

Pendant la rotation manuelle de la molette 16, l'écrou 11 est bloqué par les moyens automatiques d'entraînement 7, notamment par les moyens 9 de régulation dont le mouvement d'horlogerie est contrarié.

Lorsque le pignon moteur 18 coopére avec le pignon 11 pour entraîner automatiquement le piston 3, le fourreau 15 tend à tourner dans le sens S1 par rapport au boîtier 1, et est donc bloqué par les moyens 30 de blocage. De ce fait la vis 10 est également bloquée en rotation et se déplace axialement.

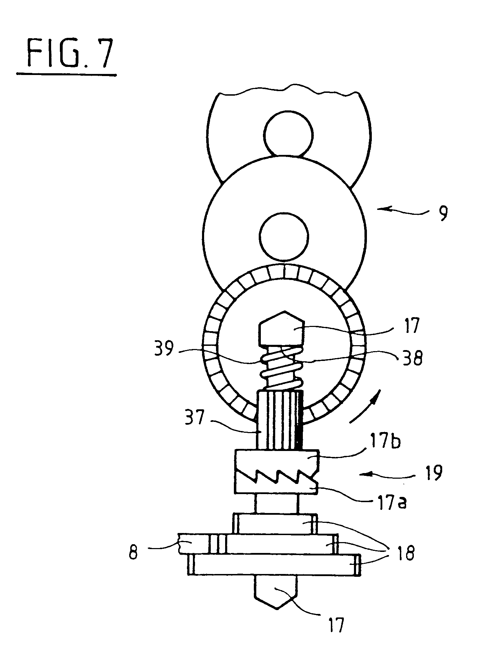

Les moyens 19 limiteurs de couple représentés en figure 7 sont constitués d'une première couronne dentée 17a solidaire de l'axe 17 et des pignons moteurs 18, et d'une seconde couronne 17b dentée qui coopére avec la première couronne 17a. les dents des couronnes 17a, 17b sont axiales, c'est-à-dire s'étendent selon leur hauteur sensiblement parallèlement à l'axe 17 et sont inclinées de façon à autoriser le glissement des couronnes 17a et 17b moyennant un déplacement axial de ces couronnes l'une par rapport à l'autre, si la couronne 17a solidaire des pignons moteurs 18 venait à se bloquer. La seconde couronne 17b porte un pignon 37 entraîné par le mouvement d'horlogerie 9. Cette couronne 17b et le pignon 37 sont traversés par l'axe 17 qui comporte à son extrémité une butée 38 coopérant avec un ressort de compression 39, qui lui, coopére avec le pignon 37 ou la couronne 17b pour rappeler cette couronne 17b contre la première couronne 17a avec une force donnée.

Lorsque le couple transmis par les deux couronnes 17a, 17b dépasse une certaine valeur, la réaction axiale sur les dents des couronnes devient égale ou supérieure à la force de rappel du ressort 39, ce qui induit le déplacement axiale de la couronne 17b et le glissement des couronnes 17a, 17b l'une par rapport à l'autre. On prévoit avantageusement des moyens d'alarme de l'utilisateur en cas de glissement des couronnes 17a, 17b l'une par rapport à l'autre. Par exemple, le déplacement axial de la couronne 17b entraîne la libération du ressort 5 des moyens de stockage, et un contacteur déclenche une sonnerie quand se ressort est totalement détendu.

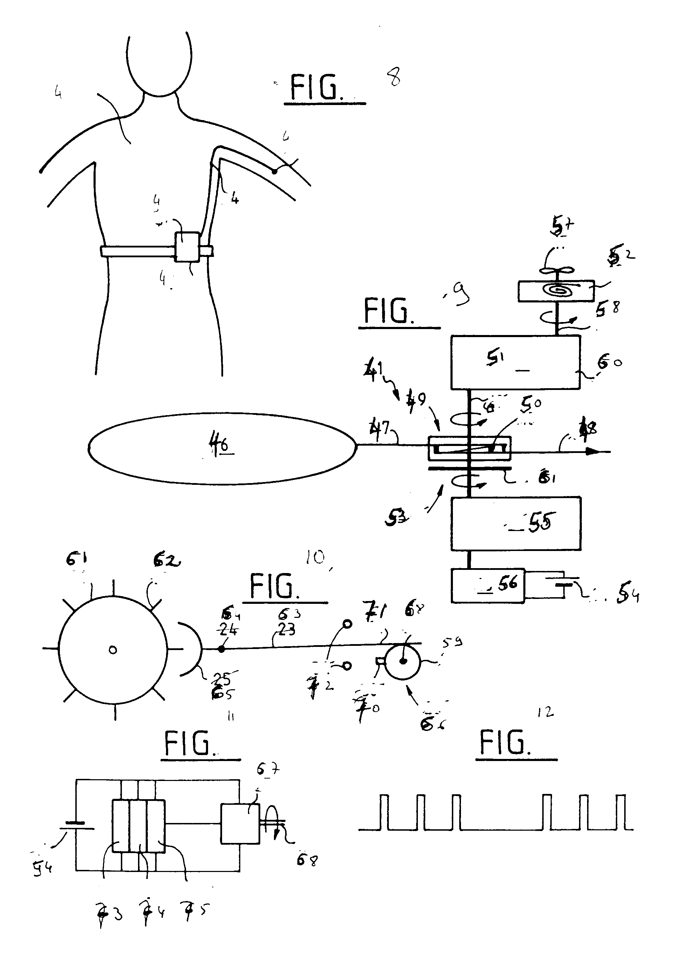

On décrira maintenant un deuxième mode de réalisation de l'invention en référence aux figures 8 à 12.

L'invention concerne un dispositif 41 , destinée à être portée par un patient 42 et à cet effet portée par un holster 43 et à laquelle est associé un cathéter 44 dont l'extrémité 45 est introduite dans une veine du patient 42 .

Le dispositif 41 est par exemple destinée à l'injection d'insuline, de produits anti-cancéreux, d'hormones ou tout autre produit approprié.

Le dispositif 41 peut être notamment du type péristaltique , bien connu en soi et ne faisant pas directement l'objet de l'invention.

Au dispositif 41 est associée, naturellement, une capacité de stockage 46 du médicament. Cette capacité 46 est constituée par exemple par une poche à fluide.

La pompe d'injection 41 comporte de façon connue en soi une entrée 47 ainsi qu'une sortie 48 destinées à être associées respectivement à la capacité 46 et au cathéter 44 ainsi que des moyens mobiles de transfert 49 interposés entre l'entrée et la sortie 47, 48. Ces moyens mobiles de transfert 49 comprennent : un organe mobile d'entraînement du médicament 50 , des premiers moyens d'entraînement 51, mobiles, associés à l'organe 50 et assurant son déplacement, et enfin une source d'énergie 52, associée aux premiers moyens d'entraînement 51 et propre à délivrer aux premiers moyens d'entraînement 51 la puissance énergétique nécessaire à leur fonctionnement et à leur entraînement.

Dans le cas où ce dispositif 41 comporte une pompe péristaltique, l'organe mobile 50 est constitué par les galets périphériques mobiles d'une roue placée dans un carter de manière qu'un tube souple reliant l'entrée 47 et la sortie 48, c'est-à-dire la capacité 46 et le cathéter 44, soit placé entre le carter et les galets pour pouvoir être compressé par les galets définissant un volume connu de médicament en déplacement.

Selon l'invention, la source d'énergie 52 est mécanique et le dispositif 41 comporte des moyens de régulation 53, électriques ou électroniques, associés aux premiers moyens d'entraînement 51 et propres à permettre le réglage du fonctionnement des premiers moyens d'entraînement 51.

Ces moyens de régulation 53 comportent en premier lieu une source d'énergie électrique 54, en second lieu des seconds moyens d'entraînement 55, mobiles, auxquels est associée la source d'énergie électrique 54 et coopérant avec les premiers moyens d'entraînement 51 et, en troisième lieu, des moyens électriques ou électroniques de commande 56 des seconds moyens d'entraînement 55.

Ainsi qu'on le verra ultérieurement, les seconds moyens d'entraînement 55 sont du type à fonctionnement tout ou rien intermittent selon un cycle de fonctionnement défini par les moyens de commande 56. Les seconds moyens d'entraînement 55 coopèrent fonctionnellement avec les premiers moyens d'entraînement 51 de manière que pendant les périodes de repos des seconds moyens d'entraînement 55, les premiers moyens d'entraînement 51 soient également maintenus au repos et que, au contraire, pendant la période de mouvement des seconds moyens d'entraînement 55, les premiers moyens d'entraînement 51 soient également en mouvement grâce à la source d'énergie mécanique 52 qui leur est associée. Comme indiqué précédemment, la succession des périodes de repos et de mouvement des seconds moyens d'entraînement 55 est déterminée par les moyens de commande 56.

La source d'énergie mécanique 52 est constituée par exemple par un ressort, notamment spiralé, pouvant être bandé au moyen d'un remontoir 57. Les premiers moyens d'entraînement 51 sont également de type mécanique et comportent notamment d'une part un axe d'entrée 58 associé au ressort 52 et d'autre part, un axe de sortie 59 associé à l'organe mobile d'entraînement de médicaments 50. Compte-tenu de la structure du dispositif 41 et des caractéristiques quantitatives usuelles pour une telle pompe, on comprend que la source d'énergie mécanique 52 doive avoir une puissance suffisante pour permettre l'entraînement de l'organe mobile 50.

Le cas échéant, et de façon connue en soi, un train d'engrenage 60 de démultiplications est interposé entre l'axe d'entrée 58 et l'axe de sortie 59.

Dans une forme de réalisation préférentielle, les seconds moyens d'entraînement 55 sont calés sur l'axe de sortie 59 des premiers moyens d'entraînement sur lequel est également calé, dans le cas d'une pompe de type péristaltique, la roue à galets précédemment mentionnée.

Les seconds moyens d'entraînement 55 peuvent faire l'objet de nombreuses formes de réalisation, selon les nécessités. En particulier, ces seconds moyens d'entraînement 55 sont de type mécanique et peuvent comporter une roue dentée 61 calée sur l'axe de sortie 59 et présentant des dents périphériques 62 régulièrement disposées autour de l'axe; un levier 63 monté pivotant autour d'un axe 64, comportant à l'une de ses extrémités une ancre 65 coopérant avec les dents 62. Des moyens 66 permettent de faire pivoter le levier 63 autour de son axe 64 de manière que, grâce à la coopération de l'ancre 65 et des dents 62 de la roue dentée 61, la roue dentée 61 effectue des pivotements successifs de la valeur angulaire séparant deux dents successives et ceci à chaque mouvement alternatif de va et vient du levier 63.

Les moyens 66 pour faire pivoter le levier 63 peuvent comporter un moteur électrique 67 de type pas-à-pas, sur l'axe de sortie 38 duquel est calée une roue 69 comportant au moins un ergot, saillie ou similaire 70 coopérant avec la partie extrême libre 71 du levier 63 opposée à l ancre 65.

Egalement, préférentiellement, il peut être prévu des butées 72 limitant l'amplitude de pivotement du levier 63 et un ressort ou similaire sollicitant le levier 63 sur la roue 69.

La source d'énergie électrique 54 peut être constituée par une pile à courant continu notamment une pile au lithium de 3 volts. En effet, ainsi que cela résulte de la description précédente et des explications qui suivent, la puissance énergétique délivrée par la source d'énergie électrique 54 est faible puisqu'elle vise à permettre uniquement le fonctionnement des moyens de régulation 53, notamment le déplacement du levier 63. En conséquence, la source d'énergie électrique 54 peut être limitée en puissance et de longue durée, notamment non remplaçable.

Les moyens de commande 56 des seconds moyens d'entraînement 55 peuvent comporter, par exemple un oscillateur 73 à fréquence réglable et un circuit de décomptage 74 susceptible de réaliser des trains d'impulsion selon des périodes déterminées et à cet effet, réglable au moyen de potentiomètres ou contacteurs à décade 75.

Grâce à un actionnement des potentiométres ou contacteurs à décade 75, il est possible de régler le train d'impulsion commandant le fonctionnement du moteur 67 donc, du levier 63 et, par conséquent, la rotation de la roue dentée 61. Lorsque la roue dentée 61 est libérée à l'occasion du mouvement de pivotement du levier 63, elle est entraînée par les premiers moyens d'entraînement 51.

Ces contacteurs comportent des moyens permettant, sans modifier un réglage de débit préalablement fixé, d'assurer le fonctionnement continu du moteur 67 aussi longtemps qu'ils sont actionnés, permettant ainsi l'administration de doses instantanées.

Selon un autre mode de réalisation, l'administration de doses instantanées est obtenue par une action manuelle directe sur l'organe mobile d'entrainement.

## Revendications

1. Dispositif externe pour injecter un médicament, notamment de l'insuline, dans le corps humain, comprenant une entrée, une sortie, des moyens mobiles de transfert interposés entre l'entrée et la sortie comprenant un organe d'entraînement (3, 50), des premiers moyens d'entraînement (6, 51) mobiles, associés à l'organe mobile et assurant son déplacement, et une source d'énergie mécanique (5, 52) associé aux premiers moyens d'entraînement et propres à délivrer la puissance énergétique nécessaire à leur fonctionnement, comportant des moyens de régulation (9, 53) associés aux premiers moyens d'entraînement (6, 51) et propres à permettre le réglage du fonctionnement des premiers moyens d'entraînement destinés à l'administration de doses continues à débit constant ou de base, caractérisé en ce qu'il comporte des moyens permettant l'administration de doses instantanées ou bolus, simultanément à l'administration desdites doses continues, grâce à des moyens mécaniques ou partiellement électriques/électroniques.

2. Dispositif selon la revendication 1, caractérisé par le fait que les moyens de stockage d'énergie (5) ont seulement pour fonction d'assurer la mise en mouvement des moyens automatiques d'entraînement (6) pour l'administration du débit de base, l'administration du bolus étant assurée seulement par l'actionnement des moyens manuels d'entraînement linéaire (7).

3. Dispositif selon la revendication 1 ou 2, caractérisé en ce que les premiers moyens d'entraînement (6) sont du type vis sans fin et écrou, dont l'écrou (11) est mobile en rotation, bloqué axialement, formant pignon mené, la vis sans fin (10) est principalement bloquée en rotation, mobile axialement par rapport à un fourreau (15) monté pivotant dans un boîtier (1) et est associée rigidement à un piston (3) par des moyens (12, 13) d'association rigide amovible, que la vis sans fin (10) est bloquée à rotation par au moins une clavette axiale (14) portée par le fourreau (15) et auquel est fixée une molette d'actionnement manuel (16), que le fourreau (15) est bloqué dans un sens de rotation, et laissé libre dans l'autre sens de rotation par rapport au boîtier (1), par des moyens (30) de blocage à cliquet anti-retour.

4. Dispositif selon l'une quelconque des revendications 1 à 3, caractérisé en ce qu'il comprend des moyens de transmission (8) comportant des organes amovibles et remplaçables de réglage de la vitesse.

5. Dispositif selon l'une quelconque des revendications 1 à 4, caractérisé par le fait que les moyens (8) de transmission comprennent un axe (17) entraîné en rotation par les moyens de régulation (9) et sur lequel est monté calé au moins un pignon moteur (18) coopérant avec le pignon mené (11) et que des moyens limiteurs de couple (19) sont interposés sur l'axe (17) en deux parties (17a, 17b) entre le pignon moteur (18) et un mouvement d'horlogerie (9).

6. Dispositif selon la revendication 5, caractérisé par le fait que plusieurs pignons moteur (18) sont montés sur l'axe (17) pour permettre le réglage de la vitesse.

7. Dispositif selon l'une quelconque des revendications 3 à 6, caractérisé par le fait que le boîtier (1) comporte deux parties montées mobiles et blocables l'une par rapport à l'autre à savoir une première partie (20) dans laquelle sont logés les moyens de stockage d'énergie (5), l'axe (17), le (ou les) pignon(s) (18) et les moyens limiteurs de couple (19), cette première partie comportant une fenêtre (22) d'accès aux dents du (ou des) pignon(s) (18) et une seconde partie (21) dans laquelle sont logés la vis sans fin (10), l'écrou (11) formant pignon mené qui saille d'une fenêtre (24), et un logement pour une seringue (2), que la seconde partie (21) comporte un logement (28) formant réservoir pour un cathéter (27), et qui comporte des moyens de rappel (26) du cathéter (27) dans ce logement (28).

8. Dispositif selon l'une quelconque des revendications 1 à 7, caractérisé en ce qu'il comporte des moyens (31) limiteurs de la dose injectée manuellement par les moyens manuels (7) d'entraînement linéaire du piston (3).

9. Dispositif selon l'une quelconque des revendications 1 à 8, caractérisé par le fait que les moyens de régulation (53) comportent en premier lieu une source d'énergie électrique (54), en deuxième lieu des seconds moyens d'entraînement (55) mobiles, auxquels est associée la source d'énergie électrique (54) et coopérant avec les premiers moyens d'entraînement (51), et, en troisième lieu, des moyens électriques ou électroniques de commande (56) des seconds moyens d'entraînement (55), que les seconds moyens d'entraînement (55) sont du type à fonctionnement tout ou rien intermittent et coopèrent fonctionnellement avec les premiers moyens d'entraînement (51) de manière que pendant les périodes de repos des seconds moyens d'entraînement (55) les premiers moyens d'entraînement (51) soient également au repos et que, au contraire, pendant les périodes de mouvement des seconds moyens d'entraînement (55), les premiers moyens d'entraînement (51) soient en mouvement grâce à la source d'énergie mécanique (52) qui leur est associée, les périodes de repos et de mouvement étant déterminées par les moyens de commande (56), que les seconds moyens d'entraînement (55) sont calés sur l'axe de sortie (59) des premiers moyens d'entraînement (51), que la source d'énergie mécanique (52) est constituée par un ressort, notamment spiralé, pouvant être bandé au moyen d'un remontoir (57), les premiers moyens d'entraînement (51) étant mécaniques, et comportant notamment d'une part, un axe d'entrée (58) associé au ressort (52) et d'autre part un axe de sortie (59) associé à l'organe mobile d'entraînement (50) du médicament.

10. Dispositif selon la revendication 9, caractérisé par le fait que les seconds moyens d'entraînement (55) comportent une roue dentée (61) calée sur l'axe de sortie (59) des premiers moyens d'entraînement (51), un levier pivotant (63) à ancre (65) coopérant avec la roue dentée (61) et des moyens (66) pour faire pivoter le levier (63) selon un mouvement alternatif de va-et-vient, que lesdits moyens (66) pour faire pivoter le levier (63), comprennent un moteur électrique (67) pas-à-pas sur l'axe de sortie (68) duquel est calée une roue (69) à ergot (70) coopérant avec ledit levier (63), que les moyens de commande (56) et les seconds moyens d'entraînement (55) comprennent un oscillateur (73) à fréquence réglable et un circuit de décomptage (74) réglable.

## Claims

1. External device to inject a medical substance, especially insulin, into the human body, said device including one inlet, one outlet, mobile transfer means inserted between the inlet and the outlet and including one actuation member (3, 50), first mobile actuation means (6, 51) associated with the mobile member and ensuring its movement, and one mechanical source of energy (5, 52) associated with the first actuation means and able to deliver the energetic power required for their functioning and comprising adjustment means (9, 53) associated with the first actuation means (6, 51) and able to adjust the functioning of the first actuation means intended to administer continuous doses with a constant or basic flow, wherein it comprises means enabling instantaneous doses or boluses be administered simultaneously with the administration of said continuous doses by means of mechanical or partially electric/electronic means.

2. Device according to claim 1, wherein the energy storage means (5) are solely provided to ensure moving of the automatic actuation means (6) so as to administer the basic dose, administration of the bolus being solely ensured via the activation of the manual means with linear drive (7).

3. Device according to claim 1 or 2, wherein the first actuation means (6) are of the endless screw or nut type whose nut (11) is rotary-mobile, axially locked and forming a driven pinion, the endless screw (10) is mainly locked in rotation and axially mobile with respect to a sheath (15) mounted pivoting inside a box (1) and is rigidly associated with a piston (3) by rigid movable association means (12, 13), wherein the endless screw (10) is locked in rotation by at least one axial key (14) borne by the sheath (15) and secured to a roller wheel with manual activation (16), wherein the sheath (15) is locked in one sense of rotation and left free in the other direction of rotation with respect to the box (1) by locking means with a nonreturn ratchet (30).

4. Device according to any one of claims 1 to 3, wherein it includes transmission means (8) comprising movable and replaceable speed adjustment members.

5. Device according to any one of claims 1 to 4, wherein the transmission means include a spindle (17) driven in rotation by the adjustment means (9) and on which at least one motor pinion (18) is mounted fixed by wedges, said pinion cooperating with the driven pinion (11) and wherein torque limiter means (19) are inserted on the spindle (17) at two portions (17a, 17b) between the motor pinion (18) and one wheel work (9).

6. Device according to claim 5, wherein several motor pinions (18) are mounted on the spindle (17) so as to be able to adjust the speed.

7. Device according to any one of claims 3 to 6, wherein the box (1) comprises two mounted mobile portions with one able to be locked with respect to the other, namely one first portion (20) housing the energy storage means (5), the spindle (17), the pinion(s) (19) and the torque limiter means (19), this first portion comprising a window (22) for gaining access to the teeth of the pinion(s) (18), and one second portion housing the endless screw (10), the nut (11) forming a driven pinion which project from one window (24), and one housing for a syringe (2), wherein the second portion (21) comprises one housing (28) forming a storage tank for a catheter (27) and which comprises means (26) for bringing back the catheter (27) into this housing (28).

8. Device according to any one of claims 1 to 7, wherein it comprises means (31) for limiting the dose injected manually by the linear drive means (7) of the piston 13).

9. Device according to any one of claims 1 to 8, wherein the adjustment means (53) firstly comprise an electric source of energy (54), secondly mobile actuation means (55) associated with the electric source of energy and cooperating with the first actuation means (51), and thirdly electric or electronic means (56) for controlling the second actuation means (55), wherein the second actuation means (55) are of the type with all or nothing functioning and functionally cooperating with the first actuation means (51) so that, during the rest periods of the second actuation means (55), the first actuation means (51) are also at rest and wherein, on the contrary, during the periods of movement of the second actuation means (55), the first actuation means (51) move by virtue of the mechanical source of energy (52) associated with them, the rest and movement periods being determined by the control means (56), wherein the second actuation means (55) are fixed by wedges to the outlet spindle (59) of the first actuation means (51), wherein the mechanical source of energy (52) is constituted by a spring, especially a spiral spring, able to be stretched by a winding-up mechanism (57), the first actuation means (51) being mechanical and comprising in particular firstly one inlet axis (58) associated with the spring (52), and secondly one outlet axis (59) associated with the mobile actuation member (50) for administering the medicine.

10. Device according to claim 9, wherein the second actuation means (55) comprise one toothed wheel fixed by wedges to the outlet axis (59) of the first actuation means (51), a swivelllng lever (63) with an anchor (65) cooperating with the toothed wheel (61) and means to make the lever (63) pivot according to an alternative to-and-fro movement, wherein said means (66) to pivot the lever (63) include one electric step motor (67) provided on the outlet axis (68) wedging a wheel (69) with a snug (70) cooperating with said lever (63), and wherein the control means (56) and the second actuation means (55) include an adjustable frequency oscillator (73) and an adjustable counting down circuit (74).

## Patentansprüche

1. Externe Vorrichtung zum Injizieren von Medikamenten, insbesondere Insulin in den menschlichen Körper, bestehend aus einen Eingang, einem Ausgang, beweglichen Fördermitteln zwischen Ein- und Ausgang mit einem Antriebsorgan (3, 50), einer ersten beweglichen Antriebsvorrichtung (6, 51) in Verbindung mit einem beweglichen Organ zu dessen Verlagerung und einer mechanischen Energiequelle (5, 52) in Verbindung mit der ersten Antriebsvorrichtung zur Entwicklung der zum Betrieb erforderlichen kinetischen Energie sowie einer Regelungsvorrichtung (9, 53) in Verbindung mit der ersten Antriebsvorrichtung (6, 51) zur Regelung von deren Betrieb, d.h. zur durchgehenden Verabreichung einer Dosis mit konstantem Durchsatz, d.h. einer Grunddosis, dadurch gekennzeichnet, daß sie Vorrichtungen besitzt, mit denen die Verabreichung momentaner Einzeldosen zusätzlich zu den durchgehend verabreichten Grunddosen mit Hilfe von mechanischen oder teilweise elektrischen bzw. elektronischen Mitteln möglich ist.

2. Vorrichtung nach Patentanspruch 1, dadurch gekennzeichnet, daß die Funktion der Mittel zur Speicherung von Energie (5) lediglich darin besteht, die automatische Antriebsvorrichtung (6) zur Verabreichung der Grunddosis in Bewegung zu setzen, während die Verabreichung der Zusatzdosis ausschließlich durch manuelles Betätigen einer linearen Antriebsvorrichtung (7) erfolgt.

3. Vorrichtung nach Patentanspruch 1 oder 2, dadurch gekennzeichnet, daß die erste Antriebsvorrichtung (6) aus einer Schnecke mit Mutter besteht, wobei die Mutter (11) sich dreht und ein getriebenes Zahnrad bildet, wahrend sie in Achsrichtung blockiert ist und die Schnecke (10) im Wesentlichen in Drehrichtung blockiert und in Achsrichtung in Bezug auf eine schwenkbar in einem Gehäuse (1) eingebauten Gleithülse (15) beweglich sowie durch eine abnehmbare Verbindungsvorrichtung (12, 13) starr mit einem Kolben (3) verbunden ist, und dadurch, daß die Schnecke in Drehrichtung durch mindestens einen axialen, von der Gleithülse (15) gehaltenen Keil (14) blockiert ist, an der ein Rad zur manuellen Betätigung (16) befestigt ist, weiterhin dadurch, daß die Gleithülse (15) in einer Drehrichtung blockiert ist, während sie sich aufgrund einer Rücklaufsperre (30) in der anderen Richtung in Bezug auf das Gehäuse (1) frei drehen läßt.

4. Vorrichtung nach einem beliebigen der Patentansprüche 1 bis 3, dadurch gekennzeichnet, daß sie eine Übertragungsvorrichtung (8) besitzt, die abnehmbare bsw. auswechselbare Organe zur Drehzahlregelung enthält.

5. Vorrichtung nach einem beliebigen der Patentansprüche 1 bis 4, dadurch gekennzeichnet, daß die Übertragungsvorrichtung (8) aus einer über eine Regelungsvorrichtung (9) in Drehrichtung angetriebenen Achse (17) besteht, auf der mindestens ein treibendes Zahnrad (18) fest angebracht ist, das in das getriebenen Zahnrad (11) eingreift, sowie dadurch, daß eine Vorrichtung zur Begrenzung des Drehmomentes (19) auf der aus zwei Teilen (17a, 17b) bestehenden Achse (17) zwischen dem treibenden Zahnrad (18) und dem Federmotor (9) angebracht ist.

6. Vorrichtung nach Patentanspruch 5, dadurch gekennzeichnet, daß mehrere treibende Zahnräder (18) aus der Achse (17) angebracht sind, um die Regelung der Drehzahl zu ermöglichen.

7. Vorrichtung nach einem beliebigen der Patentansprüche 1 bis 6, dadurch gekennzeichnet, daß das Gehäuse (1) aus zwei Teilen besteht, die zueinander beweglich und blockierbar sind, nämlich einem ersten Teil (20), in dem die Vorrichtung zum Speichern von Energie (5), die Achse (17), das bzw. die treibenden Zahnräder (18) und die Vorrichtung zur Begrenzung des Drehmomentes (19) untergebracht sind, wobei diesem erste Teil eine Öffnung besitzt, durch die die Zähne des bzw. der treibenden Zahnräder (18) zugänglich sind, sowie einem zweiten Teil (21), in dem die Schnecke (10), die das getriebene Zahnrad bildende, durch eine Öffnung (24) zugängliche Mutter (11) und eine Aufnahme für eine Injektionsspritze (2) untergebracht sind, sowie dadurch, daß das zweite Teil (21) eine Aufnahme (28) besitzt, die einen Behälter für ein Katheder (27) bildet und mit einer Rückholvorrichtung (26) zum Zurückholen des Katheders (27) in diese Aufnahme (28) versehen ist.

8. Vorrichtung nach einem beliebigen der Patentansprüche 1 bis 7, dadurch gekennzeichnet, daß sie eine Vorrichtung (31) zur Begrenzung der von Hand mit Hilfe der manuellen Vorrichtung (7) zur linearen Betätigung des Kolbens (3) injizierten Dosis besitzt.

9. Vorrichtung nach einem beliebigen der Patentansprüche 1 bis 8, dadurch gekennzeichnet, daß die Regelungsvorrichtung (53) in erster Linie aus einer elektrischen Energiequelle (54), in zweiter Linie aus einer zweiten, beweglichen Antriebsvorrichtung (55) besteht, mit der die elektrische Energiequelle (54) verbunden ist und die mit der ersten Antriebsvorrichtung (51) zusammenarbeitet, sowie in dritter Linie aus einer elektrischen bzw. elektronischen Steuervorrichtung (56) für die zweite Antriebsvorrichtung (55), sowie dadurch, daß die zweite Antriebsvorrichtung (55) im Ein/Aus-Betrieb funktioniert und so mit der ersten Antriebsvorrichtung (51) zusammenarbeitet, daß bei Rehestellung der zweiten Antriebsvorrichtung (55) die erste Antriebsvorrichtung (51) sich ebenfalls in Ruhestellung befindet und andererseits bei Betrieb der zweiten Antriebsvorrichtung (55) die erste Antriebsvorrichtung (51) aufgrund der ihr zugeordneten mechanischen Energiequelle (52) ebenfalls in Bewegung ist, wobei die Zeiten für Ruhe- bzw. Betriebsstellung von der Steurvorrichtung (56) bestimmt werden, sowie dadurch, daß die zweite Antriebsvorrichtung (55) auf der Austrittsachse (59) der ersten Antriebsvorrichtung (51) verkeilt ist, daß die mechanische Energiequelle (52) aus einer Feder besteht, insbesondere aus einer Spiralfeder, die mit einem Aufziehmechanismus (57) gespannt werden kann, vobei die erste Antriebsvorrichtung (51) mechanisch ist und insbesondere eine mit einer Feder (52) verbundene Eintrittsachse (58) und eine mit einem beweglichen Organ zum Fördern des Medikamentes verbundene Austrittsachse (59) besitzt.

10. Vorrichtung nach Patentanspruch 9, dadurch gekennzeichnet, daß die zweite Antriebsvorrichtung (55) ein auf der Austrittsachse (59) der ersten Antriebsvorrichtung (51) verkeiltes Zahnrad (61), einen mit dem Zahnrad (61) verbundenen Schwenkhebel (63) mit Anker (65) gowie eine Vorrichtung (66) zum Schwenken des Hebels (63) in abwechselnder Hin- und Herbewegung besitzt, daß diese Vorrichtung (66) zum Schwenken des Hebels (63) einen elektrischen Schrittmotor (67) auf der Austrittsachse (68) besitzt, auf der ein Rad (69) mit Nocken (70) verkeilt ist, das mit dem o.a. Hebel (63) zusammenarbeitet, und daß die Steuervorrichtung (56) und die zweite Antriebsvorrichtung (55) einen Oszillator (73) mit verstellbarer Frequenz und eine verstellbaren Rückwärtszählkreis (74) besitzen.
